Europäisches Patentamt

European Patent Office

Office européen des brevets

(11) Publication number: **0 176 112**
**B1**

# EUROPEAN PATENT SPECIFICATION

(45) Date of publication of patent specification: **16.05.90**

(21) Application number: **85200871.3**

(22) Date of filing: **03.06.85**

(51) Int. Cl.⁵: **C 12 N 15/00, C 12 N 1/20** //
(C12N1/20, C12R1:01)

(54) **Process for introducing foreign DNA into genome of plants.**

(30) Priority: **04.06.84 NL 8401780**

(43) Date of publication of application:
**02.04.86 Bulletin 86/14**

(45) Publication of the grant of the patent:
**16.05.90 Bulletin 90/20**

(84) Designated Contracting States:
**AT BE CH DE FR GB IT LI LU NL SE**

(56) References cited:
**NATURE, vol. 303, no. 5913, 12th May 1983,
pages 179-180, Macmillan Journals Ltd.,
London, GB; A. HOEKEMA et al.: "A binary plant
vector strategy based on separation of vir- and
T-region of the Agrobacterium tumefaciens
Ti-plasmid"**

**PROCEEDINGS OF THE NATIONAL ACADEMY
OF SCIENCES OF THE USA, vol. 80, no. 15,
August 1983, pages 4803-4807, Washington,
US; R.T. FRALEY et al.: "Expression of bacterial
genes in plant cells"**

(73) Proprietor: **Rijksuniversiteit Leiden
Rapenburg 73
Leiden (NL)**

(73) Proprietor: **Schilperoort, Robbert Adriaan, Prof.
Dr.
Anthonie Duycklaan 10c
NL-2334 CD Leiden (NL)**

(72) Inventor: **Hoekema, André
141 San Francisco St. App. 3
Brisbane CA 94005 (US)**
Inventor: **Hooykaas, Paul J.J.
Condorhorst 126
NL-2317 AW Leiden (NL)**
Inventor: **Hille, Jacques
de Dreijen 11
NL-6871 HG Wageningen (NL)**
Inventor: **Schilperoort, Robbert A.
Anthonie Duycklaan 10c
NL-2334 CD Leiden (NL)**

(74) Representative: **Kooy, Leendert Willem et al
OCTROOIBUREAU VRIESENDORP & GAADE
P.O. Box 266
NL-2501 AW The Hague (NL)**

Courier Press, Leamington Spa, England.

(56) References cited:
SCIENCE, vol. 222, 4th November 1983, pages 476-482, N. MURAI et al.:"Phaseolin gene from bean is expressed after transfer to sunflower via tumor-inducing plasmid vectors"

NATURE, vol. 303, 19th May 1983, pages 209-213, Macmillan Journals Ltd., London, GB; L. HERRERA-ESTRALLA et al.: "Expression of chimaeric genes transferred into plant cells using a Ti-plasmid-derived vector"

CHEMICAL ABSTRACTS, vol. 102, 1985, page 189, abstract no. 18861r, Columbus, Ohio, US; A. HOEKEMA et al.: "Delivery of T-DNA from the agrobacteriom tumefaciens chromosome into plant cells", & EMBO. J. 1984 3(11), 2485-90

## Description

The T-region of the Ti (tumour inducing)—and Ri (root inducing)—plasmid of *Agrobacterium* is transferred by nature to cells of higher plants, where this DNA is introduced into the genome and is expressed. Transfer of the T-region or any artificial T-region also occurs when this region is present in the bacterium on a separate plasmid, next to a plasmid which contains the essential virulence genes on a Vir-region (the so-called binary vector system, Dutch patent application No. 83 00698, and also mentioned as a part in a Dutch patent application for genetic manipulation of monocotyles under No. 8401048). The surprising, novel invention now described is based on an extension of the binary vector system, an intact T-region or artificial T-region being introduced into the chromosome of *Agrobacterium* as part of a transposon (Tn 1882) and a Vir-region being present on an auxiliary plasmid. The T-region, introduced into the bacterial chromosome in this way appears to be transferred to the plant cell efficiently, where it is integrated into the plant genome and is expressed. This invention also records that the binary system could also be used if the Vir-region is introduced into the bacterial chromosome and if the T-region is present on a plasmid or if the Vir-region and the T-region have been introduced into the bacterial chromosome of *Agrobacterium*. With this new invention new procedures can be developed for genetic manipulation of dicotyledonous and monocotyledonous plant cells. New possibilities for the incorporation of foreign DNA into the genome of dicotyledonous and monocotyledonous plant cells; a binary vector system that can be used in *Agrobacterium*, with which one of the components or both components are no longer present as loose replicons in the bacterium.

The invention relates to improvements in *Agrobacterium* strains suitable for genetic manipulation of higher plant cells.

A big plasmid (Ti-plasmid), present by nature in tumour provoking strains of *Agrobacterium tumefaciens* is responsible for tumour induction on dioctyledonous plants (Van Larebeke et al., Nature (London) 252, 169—170 (1974); Zaenen et al., F. Mol. Biol. 86, 109—127 (1974)). A specific part of the Ti-plasmid, called T-region, is transferred by the bacterium to the plant cell and is integrated into the core DNA (Chilton et al., Cell 11, 263—271 (1977)).

The integrated DNA is expressed (Willmitzer et al., Mol. Gen. Genet. 182, 255—262 (1981) and this expression forms the molecular base of the plant disease Crown Gall. Resulting tumour cells, in contradistinction to normal plant cells, can be grown on synthetic media without adding the plant hormones auxine and cytokinine (Braun, Proc. Natl. Acad. Sci. 44, 344:349 (1958)). The tumour cells also contain specific compounds, called opines, which are not present in normal cells and the genetic information of which lies coded on the transferred T-DNA (Bomhoff et al., Mol. Gen. Genet. 145, 177:181 (1976); Schröder et al., FEBS Lett. 129, 166—168 (1981)).

Besides the T-region, which is found back in the plant cell, the Ti-plasmid contains a second region which is essential for the virulence qualities of the bacterium (Ooms et al., J. Bacteriol. 144, 82—91 (1980) Garfinkel et al., J. Bacteriol. 144, 732—743 (1980)). This region was never seen in tumour cells and genetic analyses have shown that mutations in this area (leading to much weakened virulence or complete avirulence) are in trans complementable by wild type genes, which are present on clones or R-Prime plasmids (Hille et al. Plasmid 7, 197—118 (1982); Klee et al., J. Bacteriol. 150, 327—331 (1982)). Seven genetic loci are determined in this Vir (Virulence) region, called Vir A, B, C, D, E, F and G (Klee et al., J. Bacteriol. 150, 327—331 (1982); Hille et al. Plasmid 7, 107—118 (1982); Hooykaas et al., in press (1984)).

The Vir region and the T-region can be physically separated from one another on two compatible plasmids without any negative effect on the capacity of agrobacteria to incorporate the T-region or artificial T-region into the plant cel (Hoekema et al, Nature (London), 303, 179—180 (1983)). A binary vector system based on this invention can be used for the genetic manipulation of plant cells. (Described in Dutch patent specification No. 83 00698 and European patent application No. 84 200 239.6, and in Dutch patent application No. 84 01048).

The result presented here shows that the presence of the components of the binary system on plasmids is no essential condition. The components (T- or Vir-region) can be integrated separately or both into the chomosome of *Agrobacterium* without any negative effect on the transfer of the T-region or artificial T-region to the plant cell followed by integration into the plant genome. The invention does not only throw a vivid light on the molecular mechanism which enables the bacterium to transfer DNA to the plant cells. It also means a clear broadening of the possibilities for genetic manipulation of higher plants which are offered by the binary system.

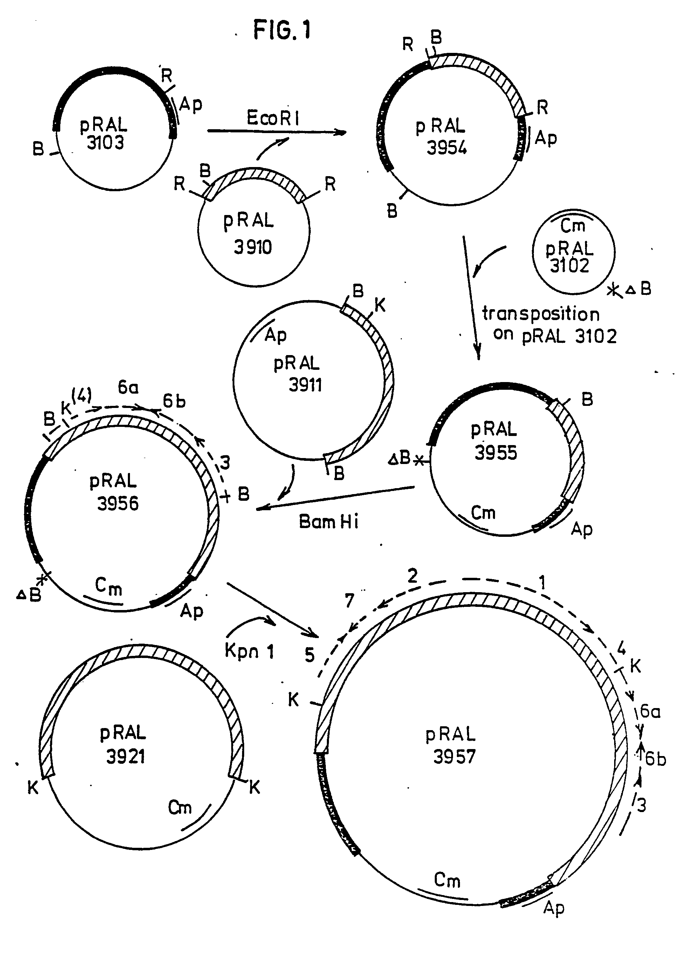

Aiming at inserting the T-region of the Ti plasmid at different locations into the genome *Agrobacterium* the transposon Tn 1882 on which the entire T-region is present was constructed *in vitro*. Three restriction enzyme-fragments which comprise the entire T-region together, viz. *Eco*RI fragment 19a, *Bam*HI fragment 17a and Kpnl fragment 9 were separately subcloned and used in a three-stage-cloning to construct the intact T-region as described hereunder, in transposon Tn3 (Δ 5—65). This transposon used (Kostriken et al., Proc. Natl. Acad. Sci 78, 4041—4045) is a derivative of Tn3 into which an EcoRI-site is introduced at a location which is not essential for transposition of the transposon or its ampicilline resistence. This transposon was further inserted into the *E. coli* vector plasmid pTR262 (Roberts et al., Gene 12, 123—127 (1980)) that even does not contain *Eco*RI site. In the unique *Eco*RI site of the resulting plasmid pRAL 3103 the *Eco*RI fragment 19a of pTiB6 was cloned. In the next stage *Bam*HI fragment 17a, which partly overlaps

with EcoRI fragment 19a, was positioned in the natural BamHl site (position 13.774 according to Barker et al., Plant. Mol. Biol. 2, 335—350 (1983)). For this purpose the Tn3 derived transposon with EcoRI fragment 19a was first transmitted to pRAL 3102, a plasmid from which the BamHl site was removed by means of in vitro deletion. To reach this plasmid pRAL 3102 was transformed to an E. coli strain where p$^{AL}$ 1155 (R 772::Tn 1880) was present. The resulting strain was used as a donor in a cross-fertilization to an empty E. coli strain. Selection for mobilisation of pRAL 3102 by pAL 1155 provided 2 types of transconjugants. The transconjugants in which Tn 1880 was leapt to pRAL 3102 were purified by isolating plasmid DNA and using this DNA again for transformation of an empty E. coli strain for the markers of pRAL 3102 and Tn 1880. Thus pRAL 3955 was obtained on which the transposon Tn 1880 is positioned. In the unique BamHl site of Tn 1880 on the plasmid 3955 BamHl fragment 17a was cloned in the correct orientation. (Vide Figure 1). Plasmid pRAL 3956 resulting therefrom does now contain the T-region genes 3, 6a, 6b and the right hand part of gene 4 as an uninterupted piece of DNA present on a transposable element. Next, the KpnI fragment 9 was cloned in the correct orientation and in the natural position (position 9834) in the unique KpnI site of pRAL 3956 lastly. The plasmid pRAL 3957 thus obtained contains the transposon Tn 1882 which comprises the intact T-region of the TiB6 plasmid.

In order to check whether Tn 1882 indeed contained a functional T-region Agrobacterium strains were constructed which contained the virulence plasmid pAL 4404 and a plasmid compatible therewith on which Tn 1882 was positioned. Therefore, Tn 1882 was inserted into plasmid R772. Plasmid R772 was cross-fertilized into an E. coli strain which contained pRAL 3957. Further cross-fertilizating to an empty strain with selection for transmission of the Ap$^R$ of Tn 1882 results in 2 kinds of transconjugants. This can be explained from the indication that plasmid R772 with low frequence pRAL 3957 could have mobilised, the other markers of pRAL being present in the transconjugants as well, or Tn 1882 could have landed on R772 by transposition. The presence of the transposon R772 in one of the 2 kinds of transconjugants was established by gelectroforesis of plasmid DNA treated with restriction enzymes. The R772 plasmid with Tn 1882 thereon was called pAL 1157. Next the plasmid pAL 1157 was brought into LBA 4404 (pAL 4404), through conjugation. The tumourigenous qualities of LBA 4404 (pAL 4404, pAL 1157) appeared to be the same as those of the wild type A. tumefaciens strain LBA 1010 for all tested host plants. This result showed that transposon Tn 1882 comprised a normally functioning T-region.

For the insertion of Tn 1882 into the chromosome of A. tumefaciens different methods have been used. A first method comprised the construction in E. coli of the plasmid pRAL 3958. This plasmid has been derived from pRK 2013, a conjugative plasmid which cannot replicate in A. tumefaciens. This means that in principle pRAL 3958 (pRK 2013::Tn 1882) can be used as "suicide plasmid" in A. tumefaciens, and then Tn 1882 can remain behind by transposition to the genome of the Agrobacterium strain, while pRAL 3958 itself gets lost. To reach this an E. coli stem with pRAL 3958 was used as a donor in a cross fertilization with A. tumefaciens stem LBA 288, which does not contain Ti plasmid.

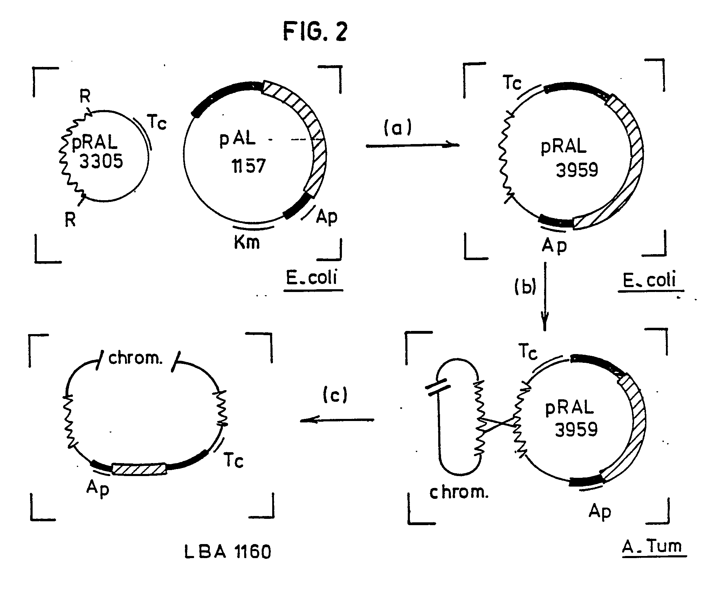

Although the resistence marker of Tn 1882 (Ap$^R$) is expressed very well in LBA 288 no transmission of the Ap$^R$ marker was found. Conjugative transmission of pRAL 3958 within E. coli occurred with a frequency of 30% per recipient. The negative result found for Agrobacterium indicates that Tn 1882 does not or hardly ever "leap" in the genome of A. tumefaciens. We avoided this problem by applying a genomic piece of DNA of A. tumefaciens to a "suicide" donor plasmid for Tn 1882 first. The strategy comprised 3 stages (vide Figure 2):

i) A. tumefaciens genomic DNA, isolated from LBA 288, was cloned in the E. coli vector pACYC 184

ii) in one of the clones, viz. pRAL 3305, Tn 1882 was introduced by means of transposition in E. coli

iii) the plasmid pRAL 3959 created this way can neither replicate in A. tumefaciens and can therefore be used for Tn 1882 in Agrobacterium as "suicide" donor.

Therefore pRAL 3959 was mobilised to LBA 288.

Now the Ap$^R$ of the Tn 1882 cannot only remain behind in Agrobacterium by possible transposition, but the entire plasmid can also come into the genome by homologous recombination over the cloned A. tumefaciens genomic DNA fragment. The mobilisation to LBA occurred with a frequency of $10^{-7}$ per recipient, while (as a check) mobilisation of this plasmid occurred within E. coli with a frequency of $10^{-4}$ per recipient. It appears from these results that pRAL 3959 can indeed not replicate in A. tumefaciens. One of the Agrobacterium transconjugants, LBA 1160, was analysed in detail in order to establish whether Tn 1882 has been introduced indeed into the chromosome of Agrobacterium.

The piece of genomic DNA (pRAL 3305) cloned from Agrobacterium strain LBA 288 might namely have originated from the chromosome or from the cryptic plasmid pAt C58 which is present in LBA 288. Therefore, it is also possible that a possible recombination of pRAL 3959 in LBA 1160 could have occurred with the chromosome and the pAtC58. Strain LBA 1160 was genetically characterized (i and ii) and the DNA of the strain was analysed by means of Southern blot hybridisation (iii) in order to be able to distinguish between both possibilities.

i) By random transposon mutagenesis the cryptic plasmid pAtC58 was provided with a selectable marker. For this purpose the "suicide" plasmid pJB4JI was used as Tn 5 donor for LBA 288, as was described for mutagenese of Rhizobium leguminosarum (Beringer et al., Nature (London), 276, 633—634)). By means of conjugation experiments it was proven that in a number of cases Tn 5 was present in LBA 288 on a self-transmissible plasmid. This plasmid appeared to be pAtC58 (transmission frequency $10^{-7}$ per recipient). This newly found quality of pAtC58 to transmit itself was used to determine whether Tn 1882 was present in LBA 1160 on pAtC58. For this purpose LBA 1160 and LBA 1201 (pAtC58::Tn5) were both used as donor strains in cross-fertilizations with LBA 285 for mutual comparison. After selection on transconjugants pAtC58::Tn 5, as was expected, appeared to be transferred with a frequency of $10^{-7}$ per recipient. However, no transmission of the $Ap^R$ of Tn 1882 could be shown. This indication formed a strong indication for the assumption that Tn 1882 was not present on the cryptic plasmid pAtC58 in the donor strain LBA 1160.

ii) Plasmids which cannot be maintained stably in one bacterium cell without selective pressure being exerted, belong to the same incompatibility class (inc.) *A fortiori* are therefore plasmids which are incompatible with themselves. If a Tn 5 marked pAtC58 ($Km^R$) is introduced into an *A. tumefaciens* strain with a Tn 1831 marked pAtC58 ($Sp^R$) and the entering plasmid ($Km^R$) is selected on, this causes a loss of the $Sp^R$ marker by incompatibility, which means removal of the plasmid already present, in the greatest part of the transconjugants. Therefore LBA 1160 is now used as recipient in a cross-fertilization with LBA 1201 (pAtC58::Tn 5) as donor. The entering plasmid pAtC58::Tn 5 was selected ($Km^R$) on and in the transconjugants it was checked whether the $Ap^R$, originating from Tn 1882, was lost by incompatibility.

It appeared that all $Km^R$ transconjugants preserved their $Ap^R$, which was again a strong indication that Tn 1882 is not positioned on pAtC58 in LBA 1160.

iii) Definite proof for the location of Tn 1882 in LBA 1160 was obtained through Southern blot hybridisation experiments, with which raw plasmid preparations of LBA 288, LBA 1201 and LBA 1160 were analysed. The raw plasmid preparations (isolated according to Casse et al., J. Gen. Microbiol. 113, 229—242 (1979) were separated in their components on agarose gels. The components which were present in the gel as bands, were transmitted direct from the gels onto nitrocellulose filters (the so-called blot procedure) and after that were hybridised with a number of specific radio-active labelled DNA probes. A controle Tn 5-probed hybridised with the so-called supercoil DNA band of pAtC58::Tn 5, and with the linear DNA band, in which besides desintegrated chromosomal DNA, desintegrated pAtC58::Tn 5 DNA of the blot is present as well. If Tn3, the starting material for Rn 1882 was used as radio-active probe, no hybridisation with pAtC58, which is present in LBA 1160, was found.

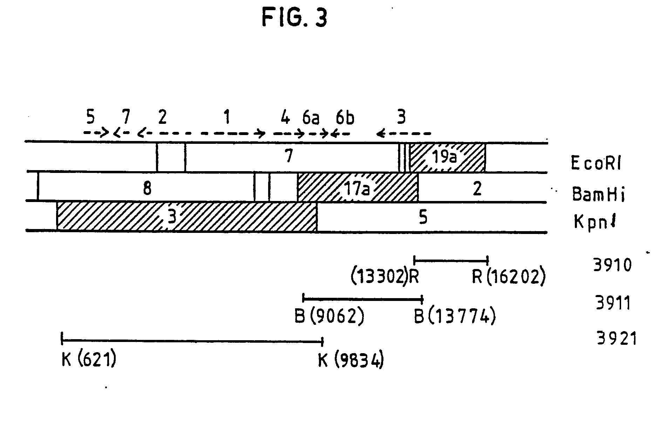

Therefore we concluded from the above that Tn 1882 and therefore the entire T-region of pTi B6 is present in the chromosome of LBA 1160. Southern blot hybridisations, which were carried out with blots on which the total DNA of LBA 1160 was present, fragmented with a number of restriction enzymes and with which pRAL 3305 and pOTY 8 were used as labelled probes confirmed the correct internal organisation of the T-region of the Tn 1882 insertion into LBA 1160. (structure as drawn in Figure 3).

The stability of the insertion of pRAL 3959 on which Tn 1882 is located, in the chromosome of LBA 1160 was examined by growing the bacteria about 100 generations without selection pressure and by checking whether the resistance markers of pRAL 3959 ($Tc^R$ and $Ap^R$) were maintained. No loss of markers was detected.

LBA 1160 therefore contains the T-region, stably introduced into the chromosome. By testing the oncogenicity of the entirely new construction obtained by us the virulence functions (Vir-region), which are necessary as well, were introduced through R-prime plasmids (pAL 1818 and pAL 1819) into the strain LBA 1160. Plasmid pAL 1818 contains the vir genes A, B, C, D, and O and pAI 1819 contains the Vir genes A up to and including F and O. After conjugation of LBA 1160 with strains which contain the R-primes and selection of transconjugants LBA 1161 (i.e. LBA 1160 (pAL 1818) and LBA 1162 (i.e. LBA 1160 (pAL 1819) were created. These strains and the parent strains LBA 1160 and LBA 1818 (pAL 1818) and LBA 1819 (pAL 1819) were tested for their tumour inducing ability on a number of different kinds of plants.

LBA 1160 and LBA 1818 and 1819 only contain one of the components of the binary system and therefore did not give rise to tumours. However, the LBA 1160 strains with a virulence plasmid (the R-primes mentioned) did surprisingly cause tumours to be created on all tested plants. Behaviour and size of the tumours entirely correspond to those of tumours induced by wild type of *Agrobacterium* strains the T-region of the Ti plasmid can therefore be normally introduced from the chromosome of *Agrobacterium* by means of Vir-region into the genome of the higher plant cell.

The observations described are very important for learning to understand the mechanism with which *Agrobacterium* transfers its T-region to the plant cell. The surprising invention that the T-region is normally transferred, even if it is present in the chromosome of *Agrobacterium,* means that it is very improbable that the entire Ti plasmid is introduced into the plant's cell during the infection and is "processed" there on behalf of integration of T-DNA, as was presumed before. Also on the ground of these results we postulate that recognition and excision of the T-DNA by virulence genes takes place within the bacterium as one of the early stages in the tumour induction process. The so-called border sequences, which border the T-region, could act as recognition signals.

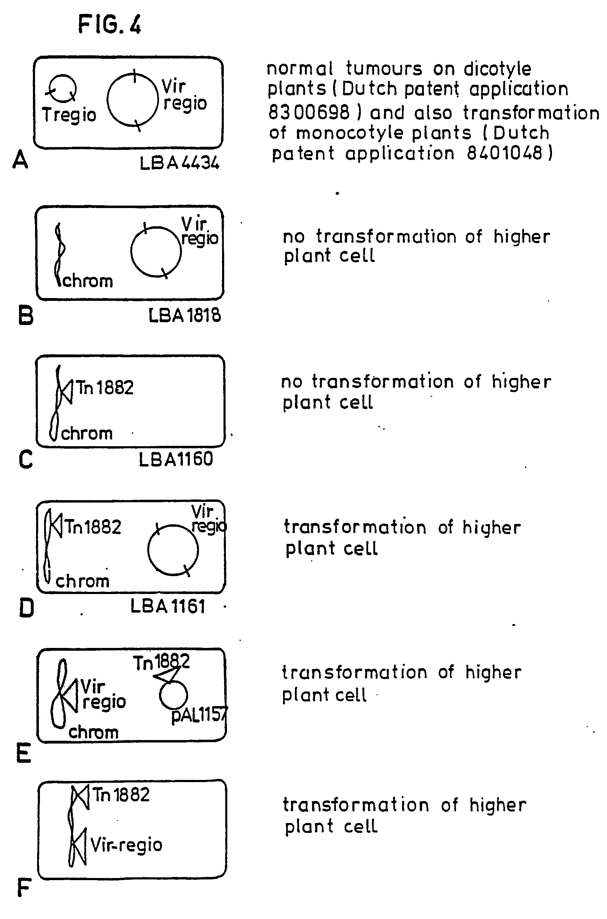

Besides this fundamental scientific importance this new invention opens new perspectives for the genetic manipulation of plant cells. The novelty of this invention and its possibilities for use can best be elucidated by means of Figure 4. A T-region and the Vir-region, which are separately present on 2 separate plasmids, are still able to transfer a T-region or an artificial T-region (and foreign DNA inserted into this) to the plant cell, where it is integrated into the genome and is expressed. With the aid of this so-called binary vector system dicotyledonous and monocotyledonous plant cells can be provided with new genetic material (Figure 4a). Strains containing only the Vir-region or only a T-region on a plasmid (Figure 4b) or in the chromosome (Figure 4c) do not cause plant cells to be transformed. With the new invention it is proved that a T-region, inserted into the chromosome, it still transferred normally to plant cells in a binary system, a Vir-region being present on a separate plasmid (Figure 4d). By this surprising invention it is also stated that if a T-region or a Vir-region or both components of Agrobacteria are not located on extrachromosomal replicons (plasmids), but are inserted into the chromosome (situations as drawn in Figures 4D, E and F) they do introduce a T-region or artificial T-region into the plant's genome in an efficient way, so that the claims as laid down in European patent application 84 200 239.6 and Dutch patent application 84 01048 can also be applied to these new situations. Such Agrobacteria can be used for the genetic manipulation of dicotyledonous and monocotyledonous plant's cells. (Dutch patent application 84 10148).

Used plasmids and bacterium stems

| Plasmids | Resistence markers | Specifications | Source |
|---|---|---|---|
| pOTY 8 | Ap | | Hirsch |
| pRAL 3101 | CmKm | pACYC 184 derivative containing unique KpnI-site | this publication |
| pRAL 3102 | Cm | pACYC 184 without BamHI site | " " |
| pRAL 3103 | ApTc | pTR 262::Tn 3 (Δ 5—65) | " " |
| pRAL 3305 | Tc | pACYC 184 with 1600 bp chromosomal EcoRI DNA fragment | " " |
| pRAL 3910 | ApEm | pBR325, cloned pTiB6 *EcoRI* fragment 19a | " " |
| pRAL 3911 | Ap | pBR313 in which cloned pTiB6 *BamHI* fragment 17a | " " |
| pRAL 3921 | Cm | pRAL 3101, in which cloned pTiB6 KpnI fragment 9 | " " |
| pRAL 3945 | ApTc | pTR262::Tn1880 | " " |
| pRAL 3955 | ApCm | pRAL 3102::Tn1880 | " " |
| pRAL 3956 | ApCm | pRAL3955 in which cloned pTiB6 *BamHI* fragment 17a | " " |
| pRAL 3957 | ApCm | pRAL3956 in which cloned pTiB6 *KpnI* fragment 9 | " " |
| pRAL 3958 | ApKm | pRK2013::Tn 1882 | " " |
| pRAL 3959 | ApTc | pRAL 3305::Tn 1882 | " " |
| pAL 1155 | ApKm | R772::Tn 1880 | " " |
| pAL 1157 | ApKm | R772::Tn 1882 | " " |
| R772 | Km | | Hedges |
| pRK 2013 | Km | | Figurski |
| KMBL 1164 | Pro⁻Thi⁻ | | vd Putte |
| HP 3435 | Bio⁻RifR | | Pannekoek |

*A. tumefaciens* stems

| | | | |
|---|---|---|---|
| LBA 285 | SpcR | | |
| LBA 288 | RifR | | |

| LBA 1010 | Rif$^R$ | pTiB6 |
|---|---|---|
| LBA 1160 | Rif$^R$ ApTc. | LBA 288; Tn 1882 in the chromosome described here |
| LBA 1161 | Rif$^R$ApTc | LBA 1160 (pAL 1818) |
| LBA 1162 | Rif$^R$ApTc | LBA 1160 (pAL 1819) |
| LBA 1201 | Km | pAtC58::Tn5. „ „ |
| LBA 1223 | KmSpc$^R$ | LBA 285 (pAtC58::Tn5). „ „ |
| LBA 1224 | Rif$^R$ApKmTc | LBA 1160 (pAtC58::Tn5). „ „ |
| LBA 1818 | | pAL 1818 |
| LBA 1819 | | pAL 1819 |
| LBA 4404 | Rif$^R$ | pAL 4404 |
| LBA 4434 | Rif$^R$ApKm | LBA 4404 (pAL 1050) |
| LBA 4440 | Rif$^R$ApKm | LBA 4404 (pAL 1157) „ „ |

## Claims

1. A process for the incorporation of foreign DNA into the genome of plants by infecting the plants or by incubating plant protoplasts with *Agrobacterium* bacteria, characterized in that *Agrobacterium* bacteria are used, which contain in their chromosome at least one T-region or a Vir-region or both, on the understanding that in the first two cases the bacteria contain a plasmid comprising a Vir-region or a T-region resp.

2. A process according to Claim 1, characterized in that *Agrobacterium* bacteria are used which contain at least one T-region having foregin DNA inserted into the T-region.

3. A process according to claim 2, characterized in that *Agrobacterium* bacteria are used which contain at least one T-region comprising only foreign DNA and flanked on both sides by border sequences as present in the wild type T-region of *Agrobacterium* bacteria.

4. *Agrobacterium* bacteria containing in their chromosomes at least one T-region or a Vir-region or both.

5. A process for producing *Agrobacterium* bacteria according to claim 4, characterized in that *E. coli* is transformed by a DNA vector containing a chromosomal DNA fragment of *Agrobacterium,* comprising at least a T-region or a Vir-region or both, which vector has the capacity to replicate in *E. coli* and which cannot survive in *Agrobacterium* bacteria per se but can integrate into their chromosomes and that the thus modified DNA-vector is introduced into the chomosomes of *Agrobacterium* bacteria.

## Patentansprüche

1. Verfahren zum Einbau von fremder DNS in das Genom von Pflanzen durch Infizieren der Pflanzen oder durch Inkubieren von Pflanzenprotoplasten mit Agrobacterium-Bakterien, dadurch gekennzeichnet, daß Agrobacterium-Bakterien angewandt werden, die in ihrem Chromosom mindestens eine T-Region oder eine Vir-Region oder beides besitzen, wobei in den ersten beiden Fällen die Bakterien ein Plasmid enthalten, umfassend eine Vir-Region bzw. eine T-Region.

2. Verfahren nach Anspruch 1, dadurch gekennzeichnet, daß Agrobacterium-Bakterien angewandt werden, die mindestens eine T-Region enthalten, wobei fremde DNS in die T-Region eingebaut ist.

3. Verfahren nach Anspruch 2, dadurch gekennzeichnet, daß Agrobacterium-Bakterien angewandt werden, die mindestens eine T-Region enthalten, umfassend nur fremde DNS und flankiert auf beiden Seiten durch Randsequenzen, wie sie in der wilden T-Region von Agrobacterium-Bakterien vorhanden sind.

4. Agrobacterium-Bakterien, enthaltend in ihrem Chromosomen mindestens eine T-Region oder eine Vir-Region oder beides.

5. Verfahren zur Erzeugung von Agrobacterium-Bakterien nach Anspruch 4, dadurch gekennzeichnet, daß E. coli durch einen DNS-Vektor transformiert ist, enthaltend ein Chromosomen-DNS-Fragment von Agrobacterium, umfassend mindestens eine T-Region oder eine Vir-Region oder beides, wobei der Vektor die Fähigkeit besitzt, sich in E. coli zu replizieren, und der in Agrobacterium-Bakterien als solchen nicht überleben kann, aber sich in ihre Chromosomen eingliedern kann, und daß der so modifizierte DNS-Vektor in die Chromosomen von Agrobacterium-Bakterien eingeführt wird.

# EP 0 176 112 B1

**Revendications**

1. Procédé d'incorporation d'ADN étranger dans le génome de plantes en infectant les plantes ou en incubant les protoplastes des plantes avec des bactéries *Agrobacterium*, caractérisé en ce qu'on utilise des bactéries *Agrobacterium* qui contiennent dans leur chromosome au moins une région T ou une région Vir ou les deux, en étant entendu que dans les deux premiers cas, les bactéries contiennent un plasmide comprenant une région Vir ou une région T respectivement.

2. Procédé selon la revendication 1, caractérisé en ce qu'on utilise des bactéries *Agrobacterium* qui contiennent au moins une région T comportant de l'ADN étranger introduit dans la région T.

3. Procédé selon la revendication 2, caractérisé en ce qu'on utilise des bactéries *Agrobacterium* qui contiennent au moins une région T comprenant seulement de l'ADN étranger et flanquée des deux côtés par des séquences marginales du type qu'on trouve dans la région T du type sauvage des bactéries *Agrobacterium*.

4. Bactéries *Agrobacterium* qui contiennent dans leur chromosome au moins une région T ou une région Vir ou les deux.

5. Procédé de production de bactéries *Agrobacterium* selon la revendication 4, caractérisé en ce qu'on transforme *E. coli* par un vecteur ADN contenant un fragment ADN chromosomique d'*Agrobacterium*, comprenant au moins une région T ou une région Vir ou les deux, vecteur capable d'une réplication dans *E. coli* et ne pouvant pas survivre dans les bactéries *Agrobacterium* par lui-même, mais pouvant s'intégrer dans leurs chromosomes et en ce que le vecteur ADN ainsi modifié est introduit dans les chromosomes des bactéries *Agrobacterium*.

## FIG. 1

## FIG. 2

# FIG. 3

# FIG. 4

A — LBA4434

normal tumours on dicotyle plants (Dutch patent application 8300698) and also transformation of monocotyle plants (Dutch patent application 8401048)

B — LBA1818

no transformation of higher plant cell

C — LBA1160

no transformation of higher plant cell

D — LBA1161

transformation of higher plant cell

E — pAL1157

transformation of higher plant cell

F

transformation of higher plant cell